# EUROPÄISCHE PATENTSCHRIFT

(11) **EP 2 564 821 B1**
(45) Veröffentlichungstag und Bekanntmachung des Hinweises auf die Patenterteilung: **30.11.2022**
(21) Anmeldenummer: 12005557.9
(22) Anmeldetag: 31.07.2012
(51) Int. Cl.: A61F 13/15

(54) **Verfahren zum Herstellen von Laminatabschnitten für medizinische Einwegartikel und Vorrichtung zur Durchführung des Verfahrens**
Procédé de fabrication de sections de laminé pour articles médicaux à usage unique et dispositif de réalisation du procédé
Method for manufacturing laminate sections for medical disposable items and device for performing the method

(30) Priorität: 03.08.2011 DE 102011109292
(43) Veröffentlichungstag der Anmeldung: 06.03.2013
(73) Patentinhaber: Paul Hartmann AG, 89504 Heidenheim (DE)
(72) Erfinder: Knecht, Theresia, 73433 Aalen (DE)

(56) Entgegenhaltungen:
- WO-A1-01/43969
- WO-A1-2010/112160
- WO-A1-2011/088099
- US-A1- 2002 103 468
- US-A1- 2004 035 521
- US-A1- 2011 146 902

## Beschreibung

Die Erfindung betrifft ein Verfahren zum Herstellen von Laminatabschnitten für medizinische Einwegartikel wie OP-Abdeckungen oder OP-Bekleidungen oder Wundabdeckungen oder absorbierende Inkontinenzprodukte.

Derartige Artikel weisen häufig eine aus einem Flachmaterial wie Vlies oder Folie gebildete erste Komponente einer ersten Länge L1 auf, auf weiche eine weitere aus einem Flachmaterial wie Vlies oder Folie oder auch Textil gebildete zweite Komponente mit einer zweiten Länge L2, wobei gilt L2 <L1, zur Bildung eines Laminatabschnittes fixiert wird, wobei die zweite Komponente vollumfänglich innerhalb der Kontur der ersten Komponente angeordnet ist.

Derartige Laminatabschnitte werden prozesstechnisch häufig derart erzeugt, dass zuvor separierte Längsabschnitte der zweiten Komponente mit der Lange L2 mittels sogenannter Cut-and-Place Vorrichtungen getaktet auf eine noch endlose Bahn der ersten Komponente appliziert und angefügt werden und erst anschließend von der Endlosbahn die Längsabschnitte des fertigen Laminatabschnittes im Wesentlichen quer zu der Längsrichtung abgetrennt werden. Das Zusammenführen von Komponenten unterschiedlicher Erstreckung in der Längsrichtung, welche der Fertigungsrichtung entspricht, ist somit prozesstechnisch anspruchsvoll.

US20110146902A1, US2002/0103468, US2004/035521A1 und WO2010/112160A1 schlagen diesbezüglich bereits vor, diejenige Endlosbahn, welche spätere Längsabschnitte der größeren Länge L1 bilden, quer zur Bahnebene und quer zur Maschinenrichtung abschnittsweise einzufalten und zu einem späteren Zeitpunkt wieder auszufalten. Darüber hinaus beschreiben WO2011/0880999A1 und WO01/43969A1 Verbunde bestehend aus einer Kombination von Vliesmaterial und Folienmaterial.

Der vorliegenden Erfindung liegt die Aufgabe zugrunde, ein Verfahren anzugeben, das in prozesstechnisch günstiger Weise und möglichst wirtschaftlich durchführbar ist.

Diese Aufgabe wird durch ein Verfahren mit den Merkmalen des Anspruch 1 gelöst.

Dadurch, dass die zweite Bahn, welche die Längsabschnitte der zweiten Komponenten der Lange L2 bilden, endlos zugeführt wird, lässt sich eine prozesstechnisch günstige und stabile Verfahrensführung realisieren, weil mit endlosen Materialbahnen bis zu deren Anfügen an die erste Bahn gearbeitet werden kann. Durch die Maßnahme des Einfaltens der ersten Bahn aus der Bahnebene in eine gewissermaßen inaktive Zwischenspeicherstellung kann die zweite Bahn endlos zugeführt und endlos an die erste Bahn angefügt werden.

Gegenstand der vorliegenden Erfindung ist außerdem eine Vorrichtung zum Herstellen von Laminatabschnitten mit den Merkmalen des Anspruchs 8.

Vorzugweise weist die zylindrische Anordnung einen walzen- oder zylinderförmigen Drehkörper auf.

Die Einfalteinrichtung und vorzugsweise auch die Fügeeinrichtung sind besonders vorteilhaft an der zylindrischen Anordnung, insbesondere an dem walzen- oder zylinderförmigen Drehkörper vorgesehen.

Besonders vorteilhaft ist, wenn die Endloseinziehbänder auf einer Kreisbahn hintereinander angeordnet sind. Nach einer ersten Variante dieses Gedankens, wird die zylinderförmige Anordnung dabei im Wesentlichen durch die Endloseinzugsbänder selbst gebildet.

Nach einer bevorzugten Ausführungsform der Anordnung der Endloseinziehbänder auf einer Kreisbahn umfasst die zylinderförmige Anordnung einen Drehkörper, wobei an dessen Mantelfläche die Endloseinziehbänder angeordnet sind. Solchenfalls bewegen sich die Endloseinziehbänder bei der Inbetriebnahme der Vorrichtung auf einer Kreisbahn.

Die Endloseinzugsbänder sind vorzugsweise auf Abstand angeordnet, so dass zwischen zwei aufeinanderfolgenden Endloseinzugsbändern überschüssiges Material der ersten Bahn aus der Bahnebene, vorzugsweise radial nach innen verbringbar ist.

Erfindungsgemäß wird das Einfalten der ersten Bahn derart ausgeführt wird, dass die erste Bahn einer zylinderförmigen Anordnung zugeführt wird, wobei die erste Bahn in der zylinderförmigen Anordnung in einem Beschleunigungsbereich mit einer Bahngeschwindigkeit v3 gefördert wird, und dass die erste Bahn danach in einem sich in Maschinenrichtung an den ersten Beschleunigungsbereich anschließenden Verzögerungsbereich auf eine zweite Bahngeschwindigkeit v5 abgebremst wird, und dass der dadurch anfallende Materialüberschuss der ersten Bahn vorübergehend durch eine Einfaltöffnung in einen jeweiligen Hohlraum der zylinderförmigen Anordnung verbracht und damit aus der Bahnebene radial verdrängt und weggefaltet wird.

Hierbei wird die erste Bahn nach der Zuführung zu der zylinderförmigen Anordnung durch ein mit der Geschwindigkeit v2 angetriebenes Endloseinzugband in dem Beschleunigungsbereich der zylinderförmigen Anordnung erfasst, wobei v2> 0 m/s ist.

Vorzugsweise wird die erste Bahn in dem Verzögerungsbereich von einem jeweiligen Endloseinzugband gefördert, das mit einer geringeren Geschwindigkeit v4 angetrieben wird als das Endloseinzugband in dem Beschleunigungsbereich (v4<v2).

Vorteilhaft ist im Falle des Vorsehens der Endloseinzugsbänder auf einem Drehkörper der Drehkörper in Betrieb mit einer ersten Bahngeschwindigkeit v1 entlang seines Umfangs (v1 entspricht also physikalisch der Tangentialgeschwindigkeit an der Mantelfläche) antreibbar. Die oben eingeführten Geschwindigkeiten v3 und v5 resultieren damit aus der Summe der Geschwindigkeiten des Drehkörpers und der Endloseinzugsbänder. Mithin gilt v3 = v1+v2 und v5 = v4+v1.

Nach eine vorteilhaften Variante dieses Verfahren beträgt v4 = 0 m/s. Das Endloseinzugband wird in dem Verzögerungsbereich also vollends angehalten und es gilt v5=v1.

Denkbar und vorteilhaft wäre hingegen auch, im Falle des Vorsehens der Endloseinzugsbänder auf einem Drehkörper, dass v4 > 0 m/s beträgt. Solchenfalls könnte die mittlere Bahngeschwindigkeit der Führung der Bahnen und damit die Produktionsgeschwindigkeit insgesamt erhöht werden, ohne die Rotationsgeschwindigkeit des Drehkörpers, welche bei gegebenem Durchmesser v1 bestimmt, über Gebühr erhöhen zu müssen.

Nach einer bevorzugten Verfahrensführung umfasst die erste Bahn ein Vliesstoffmaterial oder ist daraus gebildet. Vorzugsweise umfasst die zweite Bahn dann ein Folienmaterial oder ist daraus gebildet. Folglich umfasst die erste Komponente des Laminatabschnittes solchenfalls ein Vliesstoffmaterial oder ist daraus gebildet und die zweite Komponente des Laminatabschnittes umfasst ein Folienmaterial oder ist daraus gebildet.

Nach einer alternativen Verfahrensführung umfasst die erste Bahn ein Folienmaterial oder ist daraus gebildet. Vorzugsweise umfasst die zweite Bahn dann ein Vliesstoffmaterial oder ist daraus gebildet. Folglich umfasst die erste Komponente des Laminatabschnittes solchenfalls ein Folienmaterial oder ist daraus gebildet und die zweite Komponente des Laminatabschnittes umfasst ein Vliesstoffmaterial oder ist daraus gebildet.

Nach einer vorteilhaften Weiterbildung der Erfindung umfasst die zweite Bahn ein Körperflüssigkeiten absorbierendes Material, insbesondere ein Körperflüssigkeiten absorbierendes Vliesstoffmaterial, oder ist daraus gebildet.

Die Längsränder der zweiten Komponente sind vorzugsweise bündig zu den Längsrändern der ersten Komponente angeordnet. Die Quererstreckung (quer zur Maschinenrichtung) von erster und zweiter Komponente ist also vorzugsweise gleich.

Nach einem weiteren Erfindungsgedanken ist die Quererstreckung der zweiten Komponente geringer als die Quererstreckung der ersten Komponente.

Vorzugsweise werden durch das erfindungsgemäße Verfahren Laminatabschnitte für OP-Abdeckungen oder OP-Bekleidungen bereitgestellt.

Besonders bevorzugt werden durch das erfindungsgemäße Verfahren Laminatabschnitte für OP-Instrumententischbezüge bereitgestellt. Solchenfalls ist es besonders bevorzugt, wenn die erste Bahn einen endlosen flachgelegten Folienschlauch umfasst und die zweite Bahn aus einem Vliesstoffmaterial, insbesondere einem Körperflüssigkeiten absorbierenden Vliesstoffmaterial besteht.

Weiterhin bevorzugt werden durch das erfindungsgemäße Verfahren Laminatabschnitte für absorbierende Inkontinenzprodukte bereitgestellt. Solchenfalls ist es besonders bevorzugt, wenn die erste Bahn aus einem Folienmaterial besteht oder ein Folienmaterial umfasst und die zweite Bahn aus einer endlosen Vliesstoffmaterialbahn besteht oder diese umfasst. Solchenfalls kann die erste Komponente als die Folienkomponente und die zweite Komponente als die Vliestoffkomponente eines Backsheetlaminates für einen absorbierenden Inkontinenzartikel, insbesondere des geschlossenen Typs (Pant-type) Verwendung finden.

Denkbar und vorteilhaft ist nach einem weiteren Gedanken auch wenn die erste Bahn aus einem Vliesstoffmaterial besteht oder ein Vliesstoffmaterial umfasst und die zweite Bahn aus einem Folienmaterial besteht oder dieses umfasst. Solchenfalls kann die erste Komponente als Vliesstoffkomponente und die zweite Komponente als Folienkomponente eines Backsheetlaminates für einen absorbierenden Inkontinenzartikel, insbesondere des offenen Typs (tape-type) Verwendung finden.

Vorzugsweise kommt im Falle der Bereitstellung der Laminatabschnitte für derartige absorbierende Hygieneartikel im Zuge der Herstellung des Hygieneartikels die Folienkomponente nach innen, also körperzugewandt, und die Vliesstoffkomponente nach außen, also körperabgewandt zu liegen.

Solchenfalls ist es besonders vorteilhaft, an der noch endlosen Laminatbahn in der Längsrichtung voneinander beabstandete Saugkörperkomponenten vorzusehen, welche insbesondere auf einer Oberseite der Laminatbahn aufgebracht werden. Vorzugsweise werden die Saugkörperkomponenten hierbei so positioniert, dass die Saugkörperkomponenten vollständig innerhalb der Kontur der zweiten Komponente angeordnet ist.

Nach einer weiteren bevorzugten Variante kann die Saugkörperkomponente zwischen der ersten und der zweiten Bahn angeordnet werden, wobei die Saugkörperkomponente wiederum vorzugsweise so positioniert ist, dass die Saugkörperkomponente vollständig innerhalb der Kontur der zweiten Komponente angeordnet ist.

Nach einer Weiterbildung des Erfindungsgedankens werden an Längsrandbereichen der Laminatbahn beidseits in der Längsrichtung vorgespannte Elastifizierungsmittel, wie elastische Fäden oder elastische Bänder angeordnet und fixiert, welche insbesondere zwischen die erste Bahn und die zweite Bahn angeordnet und festgelegt werden.

Die Vliesstoffmaterialien können Fasern aus PE, PP, PET, Rayon, Cellulose, PA und Mischungen dieser Fasern enthalten. Auch Bi- oder Multikomponentenfasern sind denkbar und vorteilhaft. Vorteilhaft sind insbesondere Kardenvliese, Spinnvliese, wasserstrahlvernadelte Vliese, SM-Vliese, SMS-Vliese, SMMS- Vliese oder auch Laminatabschnitte aus einer oder mehreren dieser Vliesarten, wobei S für Spunbond- und M für Meltblown- Vliesschichten steht.

Die Vliesstoffmaterialien weisen je nach Verwendungszweck hydrophobe oder hydrophile Eigenschaften auf. Insbesondere im Falle der Verwendung erfindungsgemäß hergestellter Laminatabschnitte für die Herstellung von Instrumententischbezügen ist das Vliesstoffmaterial hydrophil und geeignet, wässrige Körperflüssigkeiten, wie Blut zu absorbieren.

Weitere Merkmale, Einzelheiten und Vorteile des erfindungsgemäßen Verfahrens und der erfindungsgemäßen Vorrichtung ergeben sich aus den beigefügten Patentansprüchen und aus der zeichnerischen Darstellung und nachfolgenden Beschreibung bevorzugter Ausführungsformen der Erfindung. In der Zeichnung zeigt:
Figur 1a eine schematische Draufsicht auf eine Inkontinenzwegwerfwindel des offenen Typs mit einem herzustellenden Laminatabschnitt;
Figur 1b einen Querschnitt entlang A-A der Figur 1a;
Figur 2a eine schematische Draufsicht auf eine Inkontinenzwegwerfwindel des geschlossenen Typs, mit einem herzustellenden Laminatabschnitt, jedoch vor der herstellerseitigen Verbindung von Rücken- und Bauchabschnitt;
Figur 2b einen Querschnitt entlang B-B der Figur 2b;
Figur 3a eine schematische Draufsicht auf einen Instrumententischbezug mit einem herzustellenden Laminatabschnitt;
Figur 3b eine Schnittansicht des über einen Instrumententisch gezogenen Instrumententischbezugs nach Figur 3a;
Figur4 eine schematische Darstellung der Zuführ-, Einfalt-, Füge-, Abtrenn- und Entfaltvorgänge nach dem erfindungsgemäßen Verfahren;
Figur 5a eine schematische Ansicht einer Vorrichtung zur Durchführung eines nicht erfindungsgemäßen Herstellungsverfahrens;
Figuren 5b und 5c schematische Ansichten von Vorrichtungen zur Durchführung des erfindungsgemäßen Herstellungsverfahrens;
Figur 6 eine Draufsicht auf einen vereinzelten Laminatabschnitt.

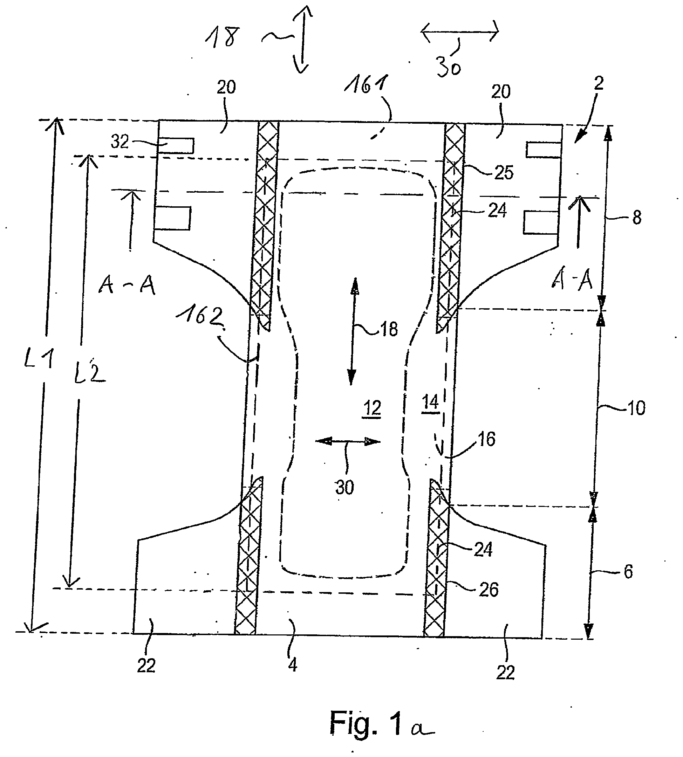

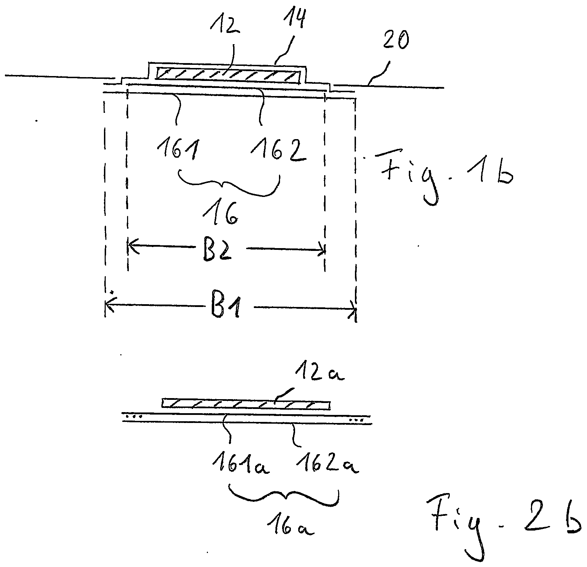

Figur 1a zeigt schematisch eine Draufsicht auf eine Innenseite, also eine körperzugewandte Seite einer öffen- und schließbaren absorbierenden Inkontinenzwegwerfwindel 2, also einer Windel des offenen Typs (tape-type), in eben ausgefaltetem Zustand. Figur 1b zeigt einen Querschnitt entlang der Linie A-A der Figur 1a. Die Inkontinenzwegwerfwindel 2 umfasst einen Hauptteil 4 mit einem Vorderbereich 6, einem Rückenbereich 8 und einem in

Längsrichtung dazwischen liegenden Schrittbereich 10. Außerdem angedeutet ist eine Saugkörperkomponente 12, der üblicherweise zwischen chassisbildenden Materialien des Hauptteils 4, also insbesondere zwischen einem flüssigkeitsdurchlässigen aus einem Vliesstoffmaterial gebildeten Topsheet 14 und einem zumindest im Bereich der Saugkörperkomponente 12 im Wesentlichen flüssigkeitsundurchlässigen Backsheet 16 des Hauptteils 4 angeordnet ist.

Das Backsheet 16 ist ein Laminatabschnitt, welcher erfindungsgemäß hergestellt ist. Der Laminatabschnitt umfasst als eine erste Komponente ein Vliesstoffmaterial 161 der Länge L1 und der Breite B1 (Erstreckung quer zur Längsrichtung L) und als eine zweite Komponente ein Folienmaterial 162 der Länge L2 und der Breite B2, wobei gilt: L2 < L1. Außerdem ist B2 < B1. Das Folienmaterial 162 befindet sich vollumfänglich innerhalb der Kontur des Vliesstoffmaterials 161.

Im Rahmen der vorliegenden Erfindung bedeutet "vollumfänglich innerhalb der Kontur", dass die zweite Komponente sich an keiner Stelle über die Kontur der ersten Komponente hinauserstreckt.

In der Verwendung des Laminatabschnittes als Backsheet 16 kommt das Vliesstoffmaterial 161 also außen und das Folienmaterial 162 innen zur Saugkörperkomponente 12 gerichtet zu liegen. Dies vermittelt der Inkontinenzwegwerfwindel 2 eine textile Anmutung. Die Saugkörperkomponente 12 liegt vollumfänglich innerhalb der Kontur der Folien-Komponente. Hierdurch ist sichergestellt, dass keine Flüssigkeit, welche in Gebrauch der Windel durch die Saugkörperkomponente 12 aufgenommen ist, nach außen durch das Backsheet 16 hindurch gelangen kann. Außerhalb der Kontur der Saugkörperkomponente 12 ist diese Gefahr naturgemäß geringer. Durch die geringere Länge L2 des Folienmaterials gegenüber der Länge L1 des Vliesstoffmaterials kann ein hohes Maß an Luftdurchlässigkeit des Hauptteils 4 sichergestellt werden.

Seitlich neben Längsrändern der Saugkörperkomponente 12 können elastische Elemente an den Hauptteil 4, insbesondere zwischen Topsheet 14 und Backsheet 16 angefügt sein (nicht dargestellt). Die elastischen Elemente würden dann vorzugsweise im Wesentlichen in Längsrichtung 18 der Inkontinenzwegwerfwindel 2 verlaufen, also mit einer wesentlichen Komponente in Längsrichtung 18, wobei sie geradlinig, oder auch einen gekrümmten Verlauf entlang des dem Schrittbereich 10 zuzuordnenden Abschnitts der Beinöffnungen nehmen können. Die Inkontinenzwegwerfwindel 2 umfasst des Weiteren hintere Seitenabschnitte 20 und vordere Seitenabschnitte 22, die als Vliesstoffkomponenten beidseits an den Hauptteil 4 angefügt sind. Die Seitenabschnitte 20, 22 sind vorzugsweise mittels Ultraschallschweißung an den Hauptteil 4 angefügt. Die Seitenabschnitte 20, 22 sind in einem schraffiert dargestellten Seitenrandbereich 24 mit chassisbildenden Materialien des Hauptteils 4, also beispielsweise mit dem Backsheet 16 und/oder dem Topsheet 14 unlösbar verbunden. Die Seitenabschnitte 20, 22 erstrecken sich über vordere und hintere Längsränder 25, 26 des Hauptteils 4 in einer Querrichtung 30 hinaus. Die hinteren seitlichen Längsränder 25, 26 des Hauptteils 4 begrenzen diejenigen Längsrandbereiche des Hauptteils, an die die Seitenabschnitte 20, 22 angefügt sind und über welche die Seitenrandabschnitte 20, 22 sich in Querrichtung hinaus erstrecken. Die Seitenabschnitte 20, 22 sind dafür gedacht und bestimmt, im angelegten Zustand der Inkontinenzwegwerfwindel 2 miteinander oder mit der Außenseite des Hauptteils 4 verbunden zu werden, um einen in Umfangsrichtung durchgehenden Hüftbereich des Hygieneartikels zu bilden. Hierzu sind an den hinteren Seitenabschnitten 20 vorzugsweise mechanische Verschlussmittel 32, insbesondere mit mechanischen Verschlusshilfen wie Kletthaken, vorgesehen, welche auf der Außenseite der vorderen Seitenabschnitte 20 und/oder auf der Außenseite des Hauptteils 4 lösbar festlegbar sind. Sowohl die vorderen Seitenabschnitte 22 als auch die hinteren Seitenabschnitte 20 sind aus einem Vliesstoffmaterial gebildet.

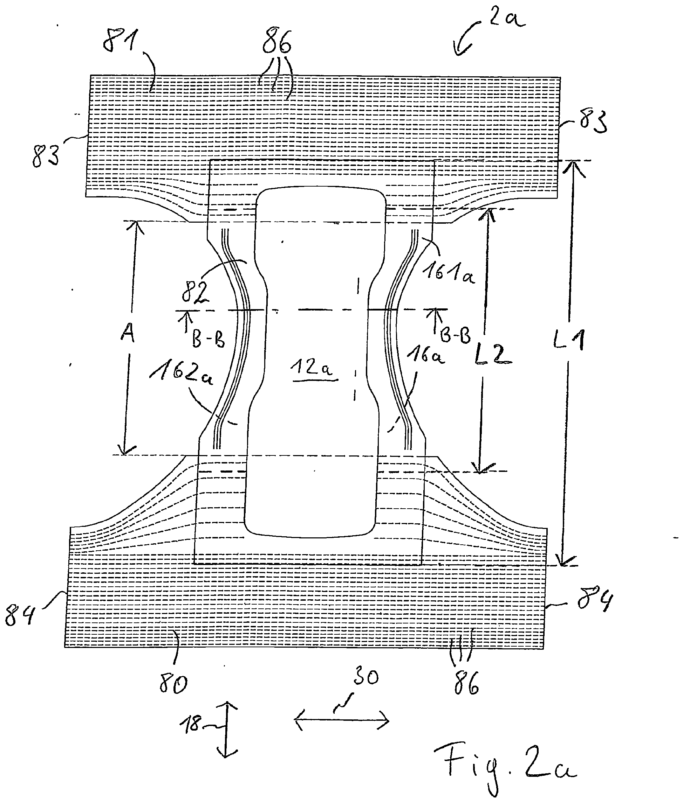

Figuren 2a und 2b zeigen einen weiteren insgesamt mit dem Bezugszeichen 2a bezeichneten Inkontinenzartikel, in Höschenform (pant-type), für die Aufnahme fester und flüssiger Körperausscheidungen. Der Inkontinenzartikel 2a ist aus drei weitestgehend unabhängig voneinander fertigbaren Komponenten, nämlich einem vorderen Bauchabschnitt 81, einem hinteren Rückenabschnitt 80 und einem zwischen diesen angeordneten und eine Saugkörperkomponente 12a aufweisenden Schrittabschnitt 82 gebildet, wobei der Schrittabschnitt 82 mit einem wesentlichen Flächenanteil des Bauchabschnitts 81 einerseits und des Rückenabschnitts 80 andererseits überlappt und im Überlappungsbereich herstellerseitig vorzugsweise unlösbar verbunden ist. Wie aus Figur 2a ersichtlich, führt dies zu einer H-förmigen Grundstruktur des Inkontinenzartikels mit einer Längsrichtung 18. Die in Figur 2a dargestellten miteinander gefügten Bestandteile werden dann zur Bildung einer nicht dargestellten geschlossenen Höschenform an jeweiligen seitlichen Längsrandabschnitten 83, 84 des Bauchabschnitts 81 und des Rückenabschnitts 80 ebenfalls herstellerseitig miteinander verbunden, wodurch beidseits in üblicher Weise Seitennahtbereiche ausgebildet werden. In diesem herstellerseitig gefertigten höschenförmigen Zustand des Inkontinenzartikels erstrecken sich der Bauchabschnitt 81 und der Rückenabschnitt 80 in Quer- oder Hüftumfangsrichtung 30 durchgehend bis zu den Seitennahtbereichen und definieren so eine in Hüftumfangsrichtung geschlossene Hüftöffnung und Beinöffnungen, durch welche hindurch der Benutzer den Inkontinenzartikel wie ein Höschen anlegt.

In dem Bauchabschnitts 81 und in dem Rückenabschnitts 80 sind Elastifizierungsmittel 86 vorgesehen, bei denen es sich insbesondere um fadenförmige Elastifizierungsmittel, wie Lycra^{®}-Fäden, handeln kann, die in vorgedehntem Zustand, im sogenannten Stretch-Bond-Verfahren, mit den Flachmaterialien (Chassismaterialien) des Bauchabschnitts 81 und des Rückenabschnitts 80 verbunden sind. Diese Elastifizierungsmittel 86 erstrecken sich in hüftöffnungsnahen Bereichen im Wesentlichen in Quer- oder Hüftumfangsrichtung 30 von einem Seitennahtbereich zum anderen. In beinöffnungsnahen Bereichen sind diese Elastifizierungsmittel hinsichtlich ihrer Orientierung bereichsweise mit einer Komponente in Längsrichtung 18 versehen, so dass die Elastifizierungsmittel gleichsam auffächern.

Der Schrittabschnitt 82 umfasst ein Topsheet (in Figuren 2a und 2b nicht dargestellt), ein Backsheet 16a das als ein Laminatabschnitt erfindungsgemäß hergestellt ist. Der Laminatabschnitt umfasst als eine erste Komponente ein Folienmaterial 161a der Länge L1 und der Breite B1 und als eine zweite Komponente ein Vliesstoffmaterial 162a der Länge L2 und der Breite B2, wobei gilt: L2 < L1. Außerdem ist im dargestellten Fall B1=B2, wobei aufgrund der sanduhrförmigen Konturierung des Schrittteils 82 die maximale Erstreckung von Folienmaterial 161a und Vliesstoffmaterial 162a in der Querrichtung 30 außerhalb der Schnittansicht B-B liegt. Das Vliesstoffmaterial 162a befindet sich vollumfänglich innerhalb der Kontur des Folienmaterials 161a, das heißt das Vliesstoffmaterial 162a erstreckt sich an keiner Stelle über den äußeren Rand des Folienmaterials 162a hinaus.

Die Saugkörperkomponente 12a liegt vollumfänglich innerhalb der Kontur des Folienmaterials 161a. Hierdurch ist sichergestellt, dass keine Flüssigkeit, welche in Gebrauch der Windel durch die Saugkörperkomponente 12a aufgenommen ist, nach außen durch das Backsheet 16a hindurch gelangen kann. In der Verwendung des Laminatabschnittes als Backsheet 16a kommt das Vliesstoffmaterial 162a auch in dieser Ausführungsform außen und das Folienmaterial 161a innen zur Saugkörperkomponente 12a gerichtet zu liegen, um der Inkontinenzwegwerfwindel 2 eine textile Anmutung zu verleihen. Da sowohl der Bauchabschnitt als auch der Rückenabschnitt bereits vorzugsweise Vliesstoffmaterialien umfassen oder daraus gebildet sind, kann die Länge L2 des Vliesstoffmaterials 162a so gering gewählt werden, dass sie den jeweiligen Abstand A zwischen den schrittzugewandten Rändern des Bauch- bzw. Rückenabschnitts gerade überbrücken kann (L2 ≥ A), so dass die Außenseite des Inkontinenzartikels vollständig durch ein Vliesstoffmaterial gebildet wird und somit der kompletten Außenseite der Windel eine textile Anmutung verliehen wird.

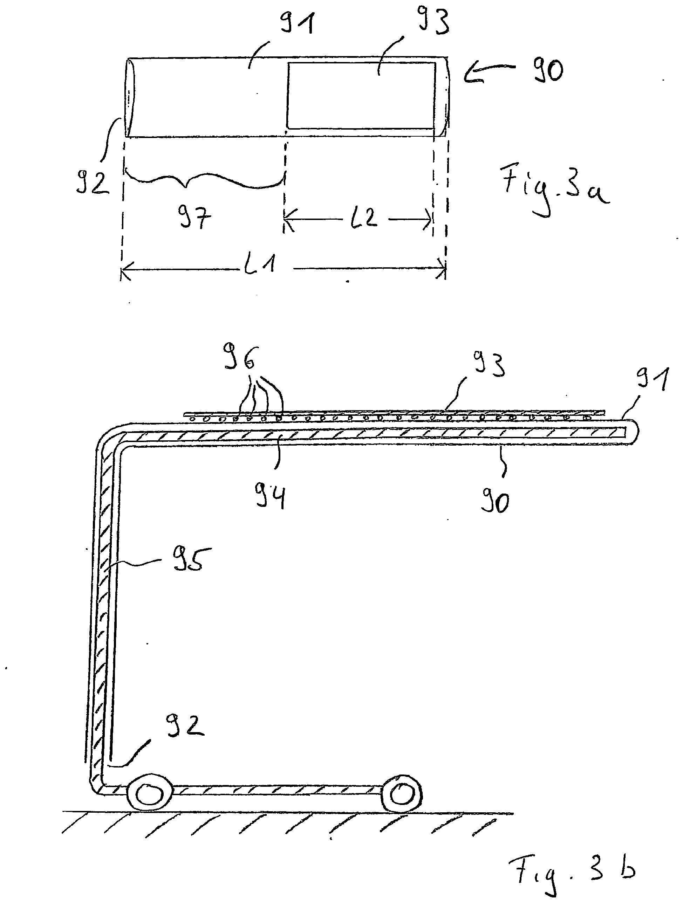

Figuren 3a und 3b zeigen einen weiteren medizinischen Einwegartikel, in Gestalt eines Instrumententischbezugs 90, welcher als ein Laminatabschnitt erfindungsgemäß hergestellt ist. Figur 3a zeigt eine Aufsicht auf die Oberseite des Instrumententischbezuges 90. Der Instrumententischbezug 90 umfasst als erste Komponente einen an drei Seiten geschlossenen Folienschlauchabschnitt 91 der Länge L1. Mit dem offenen Ende 92 kann der Instrumententischbezug über einen Instrumententisch 95 gezogen werden (Figur 3b). Zur Vereinfachung dieses Vorgangs kann der Instrumententischbezug mit einer in DE10102001A1 beschriebenen innenliegenden Stülpfaltung versehen sein. Auf seiner Oberseite weist der Instrumententischbezug 90 als zweite Komponente ein wässrige Flüssigkeiten absorbierendes Vliesstoffmaterial 93 der Länge L2 auf, wobei gilt L2 < L1. Der Instrumententischbezug 90 ist in Figur 3b über eine sich flächenhaft erstreckende Tischplatte 94 eines OP-Instrumententisches 95 gezogen. In dieser Ansicht ist erkennbar, dass die Länge L2 des Vliesstoffmaterials 93 so ausgelegt ist, dass die Oberseite der Längserstreckung der Tischplatte 94 und damit die für das OP-Personal zur Verfügung stehende Ablagefläche für OP-Instrumente im Wesentlichen gänzlich durch das Vliesstoffmaterial 93 überfangen ist, welches im dargestellten Fall vermittels eines diskontinuierlich vorgesehenen Heißschmelzklebers 96 flächenhaft an dem Folienschlauchabschnitt 91 angefügt ist. An den Instrumenten nach Gebrauch - auch nach zwischenzeitlichem Gebrauch - abtropfende Flüssigkeiten wie Blut oder OP-Spülflüssigkeiten können so von dem Vliesstoffmaterial 93 des Instrumententischbezuges 90 absorbiert werden. Ein Endabschnitt 97 des Instrumententischbezugs 90, welche in Gebrauch im Wesentlichen lediglich über einen unteren Teil des Instrumententisches 95 gezogen ist, kann Vliesstoffmaterial-frei verbleiben.

Nachfolgend werden Varianten des Verfahrens und der Vorrichtung zur Herstellung vorstehend beschriebener Laminatabschnitte näher erläutert:

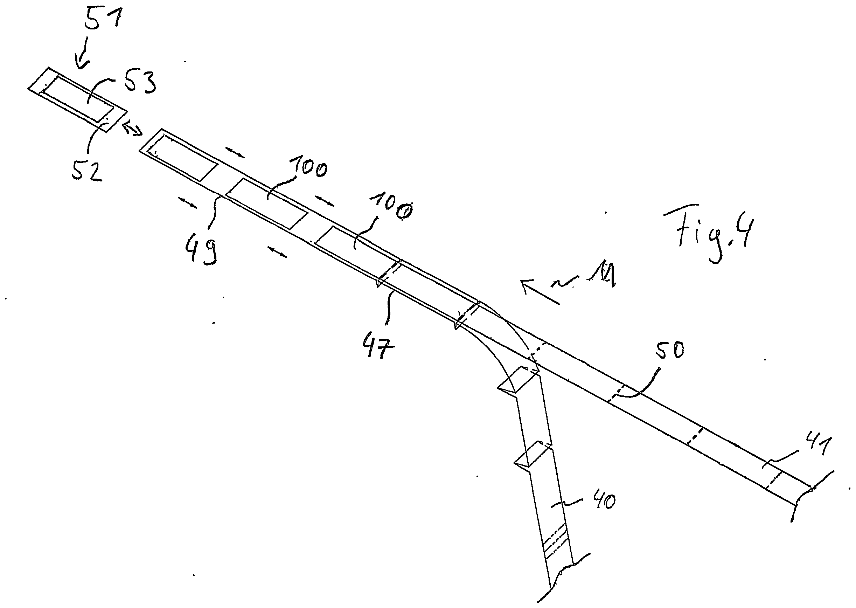

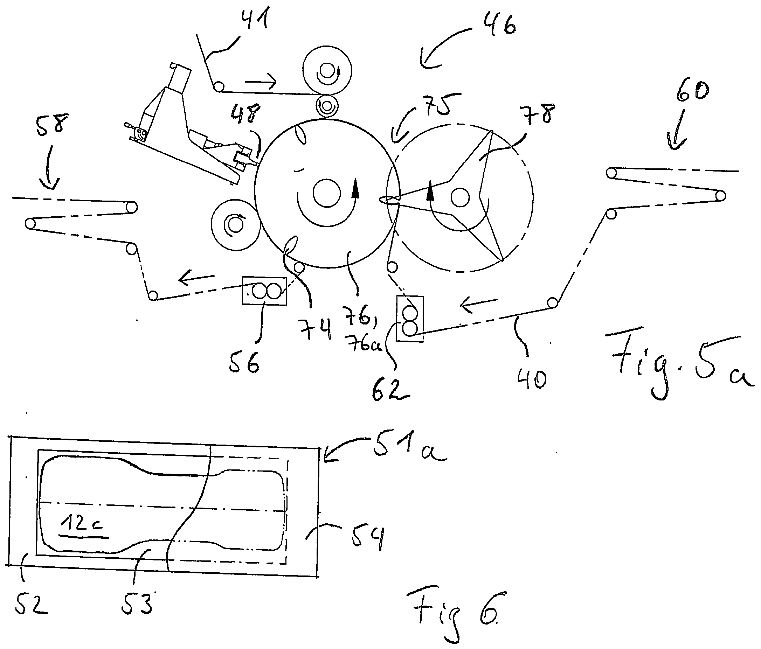

Figuren 4 und 5a (nicht erfindungsgemäß) und 5b zeigen schematisch die verschiedenen hier im Mittelpunkt des Interesses stehenden Schritte bei der Herstellung der Laminatabschnitte 51. Es wird eine erste endlose Bahn 40 eines ersten Flachmaterials, mit einer ersten Breite B1 (Erstreckung quer zur Maschinenrichtung M) endlos in Maschinenrichtung M zugeführt, welche die ersten Komponenten 52 der späteren Laminatabschnitte 51 bilden. Ebenso wird eine zweite endlose Bahn 41 eines zweiten Flachmaterials mit einer zweiten Breite B2 endlos in Maschinenrichtung M zugeführt, welche die zweiten Komponenten 53 der späteren Laminatabschnitte 51 bilden. Im dargestellten Fall (Figur 4) ist B2<B1. Das Material der zweiten endlosen Bahn 41 kann bereits offline, zum Beispiel im Zuge der Herstellung der endlosen Bahn 41 mit einer Abfolge von quer zur Maschinenrichtung angeordneten Schwächungslinien 50, wie insbesondere einer Perforation versehen sein. Der Abstand einer Perforation zur benachbarten Perforation in Maschinenrichtung M entspricht im Wesentlichen der Länge L2 der zweiten Komponenten 53 eines späteren Laminatabschnittes 51. Die erste Bahn 40 könnte auch inline, beispielsweise unmittelbar vor den nachfolgend beschriebenen Verfahrensschritten mit diesen Schwächungslinien 50 versehen werden.

Bevor die zweite endlose Bahn 41 an der ersten endlosen Bahn 40 appliziert und angefügt wird, wird die erste Bahn 40 quer zur Maschinenrichtung M und senkrecht zur Bahnebene eingefaltet.

Figur 5a zeigt eine nicht erfindungsgemäße Vorrichtung 46 zur Durchführung des Verfahrens, wobei Einfaltöffnungen 74 bei einer Einfalteinrichtung 75 mit einem eine zylinderförmige Anordnung 76 bildenden ersten Drehkörper 76a und einem drehenden Verdrängerorgan 78 der Vorrichtung 46 für eine entsprechend tiefe Einfaltung der ersten Bahn 40 ausgebildet sind. Die Einfalttiefe T ist dabei derart gewählt, dass im Wesentlichen die gesamte Länge des von der zweiten Komponente 53 freien Bereichs des späteren Laminatabschnittes aus der Bahnebene verdrängt und weggefaltet ist. Die Einfalttiefe T beträgt dabei vorzugsweise 5-70, weiter vorzugsweise 10-60 cm. Die zweite Bahn 41 wird dann im dargestellten Fall zentriert mittig, jedenfalls innerhalb der Längsränder der ersten Bahn 40, auf die erste Bahn 40 appliziert und durch Vorbeiführen an einer als Ultraschallsonotrode ausgebildeten Fügeeinrichtung 48 unlösbar mit der ersten Bahn 40 verbunden, und zwar in Maschinenrichtung M im Wesentlichen durchgehend (auch wenn einzelne Schweißpunkte hierfür Verwendung finden). Denkbar und vorteilhaft wäre auch, die Unterseite der zweiten Bahn unmittelbar vor dem Zusammenführen der Bahnen mit einem Haftmittel wie etwa einem Kleber, insbesondere einem Hotmeltkleber zu versehen und die Bahnen dann beispielsweise mithilfe einer Andruckrolle zu verbinden. Auch das Verbinden der Bahnen mittels anderer Fügeverfahren wie Thermoschweißen, insbesondere vermittels entsprechender Kalanderwalzen ist denkbar und vorteilhaft.

Die so hergestellte Vor-Laminatbahn 47 könnte zunächst endlos aufgerollt und erst zu einem späteren Zeitpunkt im Zuge der Herstellung von medizinischen Einwegartikeln den weiteren Verfahrensschritten zugeführt werden.

Vorteilhaft ist es jedoch, den Verbund unmittelbar im Anschluss an die Verbindung der Bahnen vermittels einer Abzugseinrichtung 56 zu beschleunigen und dabei zu entfalten. Dabei werden spätere zweite Komponenten 53 bildende, aufeinander folgende Längsabschnitte 100 der Bahn 41 entlang der Schwächungslinie 50 getrennt und voneinander beabstandet. Gleichzeitig wird der eingefaltete Abschnitt wieder in die Bahnebene gebracht also entfaltet. Zur Aufnahme dieser eingefalteten Länge ist eine Tänzeranordnung 58 der Abzugseinrichtung 56 nachgeordnet. Eine entsprechende Tänzeranordnung 60 und im dargestellten Fall als S-Wrap ausgeführte Zuführeinrichtung 62 ist dem vorstehend beschriebenen Prozess vorgeordnet.

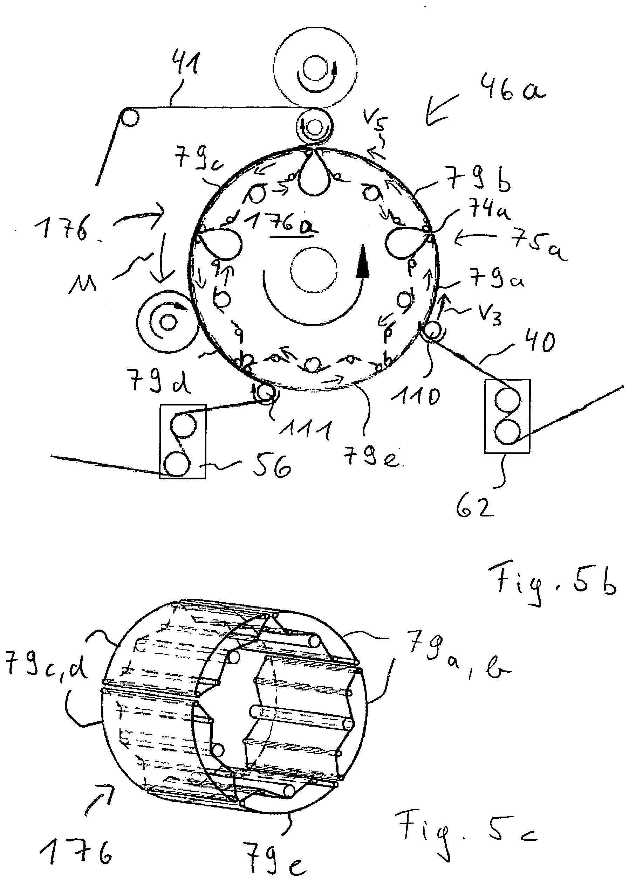

Figur 5b zeigt eine Vorrichtung 46a zur Durchführung des erfindungsgemäßen Verfahrens. Die Vorrichtung 46a umfasst eine zylinderförmige Anordnung 176 mit einem walzenförmigen Drehkörper 176a, an dessen Mantelfläche und damit dessen Mantelfläche im Wesentlichen bildend eine Mehrzahl von - im dargestellten Fall 5 - antreibbaren Endloseinziehbändern 79a,b,c,d,e angeordnet ist, wobei jedes Endloseinziehband sich vorzugsweise über einen gleich großen Umfangsabschnitt des Drehkörpers 176a erstreckt. Figur 5c zeigt in einer perspektivischen, schematischen Ansicht die Anordnung der Endloseinziehbänder 79 a, b, c, d, e mitsamt ihrer Führungswalzen. Der Übersicht halber zeigt die Ansicht der Figur 5c keine weiteren Bauteile der zylindrischen Anordnung 176.

Die Endloseinziehbänder bilden die Einfalteinrichtung 75a. Der Drehkörper 176a wird im Betrieb in Drehbewegung versetzt, so dass eine erste Bahngeschwindigkeit v1 entlang seiner Mantelfläche (Tangentialgeschwindigkeit) resultiert. Die Endloseinziehbänder 79a,b,c,d,e sind in Umfangsrichtung des Drehkörpers 176a voneinander geringfügig beabstandet, mithin auf Lücke angeordnet. Wie bei der in Figur 5a dargestellten Vorrichtung wird über eine im dargestellten Fall als S-Wrap ausgeführte Zuführeinrichtung 62 die erste Bahn der zylindrischen Anordnung 176 zugeführt. Über eine Rolle 110 gelangt die Bahn 40 innerhalb eines ersten Beschleunigungsbereichs der zylindrischen Anordnung 176 auf ein erstes Endloseinzugband 79a des Drehkörpers 176a. Die Rolle 110 ist im in Figur 5b dargestellten Fall als Antriebsrolle für ein jeweiliges Endloseinzugband ausgebildet und treibt es mit v2 (relativ zu V1) an, sobald ein jeweiliges Endloseinziehbänder von der Rolle 110 erfasst ist. V2 und v1 addieren sich zu Bahngeschwindigkeit v3, mit der die erste Bahn 40 in dem ersten Beschleunigungsbereich auf dem Drehkörper 176a gefördert wird.

In einem sich in Maschinenrichtung M daran anschließenden Verzögerungsbereich wird das erste Endloseinzugband nachfolgend abgebremst auf v4, so dass sich die Bahngeschwindigkeit v5 der ersten Bahn in dem Verzögerungsbereich mit v4+v1 berechnet. Im dargestellten Fall resultiert der Verzögerungsbereich dadurch, dass das jeweilige Einzugsband dem Einwirken der Rolle 110 gänzlich entzogen ist, so dass dann eine Relativbewegung des Endloseinzugbandes gegenüber dem Drehkörper 176a unterbleibt. V4 ist somit im dargestellten Fall gleich 0 m/s und damit v5 = v1. Aufgrund der Geschwindigkeitsdifferenz v3-v5 wird in Längsrichtung überschüssiges Material der ersten Bahn 40 vorübergehend über eine hierfür vorgesehene jeweilige Einfaltöffnung 74a in einen Hohlraum innerhalb des Drehkörpers 176a zwischen die Lücke zweier aufeinanderfolgender Endloseinzugbänder verbracht und damit aus der Bahnebene im wesentlichen radial verdrängt und weggefaltet. Wie im Zusammenhang mit Figur 5a beschrieben wird anschließend die zweite Bahn 41 auf die erste Bahn 40 appliziert und durch Vorbeiführen an einer Ultraschallsonotrode (in Figur 5b nicht dargestellt) unlösbar mit der ersten Bahn 40 verbunden. Die so hergestellte Vor-Laminatbahn könnte zunächst endlos aufgerollt und erst zu einem späteren Zeitpunkt im Zuge der Herstellung von medizinischen Einwegartikeln den weiteren Verfahrensschritten zugeführt werden. Bevorzugt jedoch wird der Verbund unmittelbar im Anschluss an die Verbindung der Bahnen in einem zweiten Beschleunigungsbereich vermittels einer Rolle 111 beschleunigt auf eine Bahngeschwindigkeit v7, die Im Wesentlichen v3 entspricht. Die Rolle 111 ist im dargestellten Fall als Antriebsrolle für ein jeweiliges Endloseinzugband ausgebildet und treibt es mit v6 an, sobald ein jeweiliges Endloseinziehbänder von der Rolle 111 erfasst ist, so dass sich die Bahngeschwindigkeit v7 der ersten Bahn in dem zweiten Beschleunigungsbereich mit v6+v1 berechnet. Durch die Beschleunigung der Bahn werden spätere zweite Komponenten 53 bildende, aufeinander folgende Längsabschnitte 100 der Bahn 41 entlang der Schwächungslinie 50 getrennt und voneinander beabstandet (siehe Figur 4). Gleichzeitig wird der eingefaltete Abschnitt wieder in die Bahnebene gebracht also entfaltet. Der entfaltete Verbund wird mittels einer als S-Wrap ausgebildeten Abzugseinrichtung 56 vorzugsweise einer Tänzeranordnung zugeführt, welche geeignet ist, die Spannung der Laminatbahn 49 aufrechtzuerhalten.

Während die in Figur 5a dargestellte nicht erfindungsgemäße Vorrichtung 46 eine periodische Änderung sowohl der Zuführgeschwindigkeit der ersten Bahn 40 als auch der Abzuggeschwindigkeit der Laminatbahn 49 erfordert, ermöglicht die in Figur 5b dargestellte erfindungsgemäße Vorrichtung 46a, die Zuführ- und Abzugsgeschwindigkeit jeweils im Wesentlichen konstant zu halten. Gleichwohl wäre es denkbar, die oben bezeichneten Geschwindigkeiten v1, v2, v3, v4, v5, v6 oder v7 im Betrieb periodisch oder nicht periodisch veränderlich zu führen.

Die Vorrichtung nach Figur 5b gestattet außerdem ein hohes Maß an Variabilität bezüglich der Einfalttiefe, und damit bezüglich des Verhältnisses der Längen L1 und L2, da die Einfalttiefe innerhalb physikalischer Grenzen lediglich durch Änderungen des Verhältnisses v3/v5 einstellbar ist. Das Verhältnis v3/v5 beträgt vorzugsweise 1:0,95 bis 1:0,1 insbesondere 1:0,9 bis 1:0,3.

Nach der in Figur 5b dargestellten Vorrichtung sind die Endloseinzugbänder auf einem rotierbaren Körper (Drehkörper) angeordnet.

Die nach einem der beschriebenen Verfahren hergestellte Laminatbahn 49 könnte zunächst endlos aufgerollt und erst zu einem späteren Zeitpunkt im Zuge der Herstellung von medizinischen Einwegartikel dem weiteren Verfahrensschritt der Vereinzelung zugeführt werden.

Vorzugsweise werden jedoch unmittelbar im Anschluss an den Entfaltungsvorgang Laminatabschnitte 51 von der Laminatbahn 49, vorzugsweise vermittels rotierender Messerwalzen abgetrennt und dem Herstellprozess medizinischer Einwegartikel zugeführt. In Figur 4 ist ein derartiger Laminatabschnitt 51 bestehend aus einer ersten Komponente 52 einer ersten Länge L1, welche aus der ersten Bahn 40 gebildet ist, und einer zweiten Komponente 53 mit einer zweiten Länge L2, welche aus der zweiten Bahn 41 gebildet ist wobei gilt L2 < L1, und wobei die zweite Komponente vollumfänglich innerhalb der Kontur der ersten Komponente angeordnet ist, dargestellt.

Weiterhin denkbar wäre im Falle der Verwendung des Laminatabschnittes 51 als Schrittabschnitt oder Hauptteil einer Inkontinenzwegwerfwindel wie zuvor in Figuren 1a und 2a beschrieben, auf die Laminatbahn und zeitlich entweder unmittelbar im Anschluss an den oder kurz vor dem Entfaltungsvorgang, Absorptionskörper (das heißt zur dauerhaften Aufnahme und Speicherung von Körperflüssigkeiten wie Urin bestimmte Komponenten) getaktet innerhalb der Kontur der zweiten Komponente vorzusehen und dort anzufügen. Vorzugsweise könnte daran anschließend, jedenfalls nach dem Entfaltungsvorgang, die Laminatbahn oberhalb der Absorptionskörper mit einer endlosen Topsheetbahn versehen werden, welche die gleichen Abmessungen wie die entfaltete erste Bahn aufweist, derart, dass Topsheetbahn und die erste Bahn den Absorptionskörper sandwichartig einschließen. Einen derartigen Laminatabschnitt 51a, mit einer ersten Komponente 52 und einer zweiten Komponente 53, einer Saugkörperkomponente 12c und einem Topsheetmaterial 54 (dargestellt im rechten Teil der Figur) zeigt Figur 6.

Im Falle der Verwendung des Laminatabschnittes 51 als Schrittabschnitt oder Hauptteil einer Inkontinenzwegwerfwindel bestehen die erste und die zweite Komponente vorzugsweise aus einem Folienmaterial oder einem Vliesstoffmaterial. Im Falle der Verwendung des Laminatabschnittes 51 als Instrumententischbezug umfasst die erste Komponente vorzugsweise einen flachgelegten Folienschlauch und die zweite Komponente ein Körperflüssigkeiten absorbierendes Vliesstoffmaterial.

## Patentansprüche

1. Verfahren zur Herstellung von Laminatabschnitten (51) für medizinische Einwegartikel (2, 2a, 90) wie absorbierende Inkontinenzprodukte oder OP-Abdeckungen oder OP-Bekleidungen oder Wundabdeckungen, wobei die Laminatabschnitte (51) eine aus einem Flachmaterial wie Vlies oder Folie gebildete erste Komponente (52) einer ersten Länge L1 und eine weitere aus einem Flachmaterial wie Vlies oder Folie gebildete zweite Komponente (53) mit einer zweiten Länge L2 umfassen, wobei gilt L2 < L1, und wobei die zweite Komponente (53) vollumfänglich innerhalb der Kontur der ersten Komponente (52) angeordnet ist, und wobei eine erste endlose Bahn (40) eines ersten Flachmaterials in einer Längsrichtung, die der Maschinenrichtung M entspricht, gefördert wird, und wobei eine zweite Bahn (41) eines zweiten Flachmaterials in der Längsrichtung gefördert wird und wobei die zweite Bahn (41) im Wesentlichen quer zu der Längsrichtung angeordnete Schwächungslinien (50) aufweist oder die Schwächungslinien (50) im Zuge der Herstellung der Laminatabschnitte (51) erzeugt werden, und die erste Bahn (40) quer zur Bahnebene und quer zur Maschinenrichtung M eingefaltet wird, und wobei nach diesem Einfalten der ersten Bahn (40) die zweite Bahn (41) auf die erste Bahn (40) appliziert und angefügt wird, derart, dass die zweite Bahn (41) innerhalb der Längsränder der ersten Bahn (40) angeordnet ist, und dann der Verbund aus der ersten Bahn (40) und der zweiten Bahn (41) zur Bildung einer Laminatbahn (49) in Maschinenrichtung M beschleunigt und dabei entfaltet wird, wobei die zweite Bahn (41) entlang der Schwächungslinien (50) getrennt und dadurch Längsabschnitte (100) der zweiten Bahn voneinander beabstandet werden und wobei zur Bildung der Laminatabschnitte (51) die Laminatbahn (49) im Wesentlichen quer zur Maschinenrichtung M vereinzelt wird, **dadurch gekennzeichnet, dass** das Einfalten der ersten Bahn derart ausgeführt wird, dass die erste Bahn (40) einer zylinderförmigen Anordnung (176) zugeführt wird, wobei die erste Bahn in der zylinderförmigen Anordnung (176) in einem ersten Beschleunigungsbereich mit einer Bahngeschwindigkeit v3 gefördert wird, und dass die erste Bahn danach in einem sich in Maschinenrichtung an den ersten Beschleunigungsbereich anschließenden Verzögerungsbereich auf eine zweite Bahngeschwindigkeit v5 abgebremst wird, und dass der dadurch anfallende Materialüberschuss der ersten Bahn (40) vorübergehend in einen jeweiligen Hohlraum unterhalb einer Einfaltöffnung (74a) der zylinderförmigen Anordnung verbracht und damit aus der Bahnebene radial verdrängt und weggefaltet wird, und dass die erste Bahn (40) nach der Zuführung zu der zylinderförmigen Anordnung (176) durch ein mit der Geschwindigkeit v2 angetriebenes Endloseinzugband (79a) in dem ersten Beschleunigungsbereich der zylinderförmigen Anordnung (176) erfasst wird, wobei v2> 0 m/s ist..

2. Verfahren nach Anspruch 1, **dadurch gekennzeichnet, dass** ein die erste Bahn (40) förderndes Endloseinzugband (79b) in dem Verzögerungsbereich mit einer geringeren Geschwindigkeit angetrieben wird als das Endloseinzugband (79a) in dem ersten Beschleunigungsbereich, so dass die erste Bahn (40) abgebremst wird.

3. Verfahren nach Anspruch 1 oder 2, **dadurch gekennzeichnet, dass** die zylinderförmige Anordnung (176) einen zylinderförmigen Drehkörper (76a, 76b) umfasst, an dessen Umfängsfläche die Endloseinzugbänder (79a, 79b, 79c, 79d, 79e) angeordnet sind.

4. Verfahren nach einem der Ansprüche 1-3, **dadurch gekennzeichnet, dass** a.) die erste Bahn (40) ein Vliesstoffmaterial umfasst oder daraus gebildet ist und die zweite Bahn (41) ein Folienmaterial umfasst oder daraus gebildet ist oder b.) die erste Bahn (40) ein Folienmaterial umfasst oder daraus gebildet ist und die zweite Bahn (41) ein Vliesstoffmaterial umfasst oder daraus gebildet ist.

5. Verfahren nach einem der vorstehenden Ansprüche, **dadurch gekennzeichnet, dass** die zweite Bahn (41) ein Körperflüssigkeiten absorbierendes Material umfasst oder daraus gebildet ist.

6. Verfahren nach einem der vorstehenden Ansprüche, **dadurch gekennzeichnet, dass** in den Laminatabschnitten (51) die Längsränder der zweiten Komponente (53) bündig zu den Längsrändern der ersten Komponente (52) angeordnet sind.

7. Verfahren nach einem der vorstehenden Ansprüche, **dadurch gekennzeichnet, dass** an der Laminatbahn (49) in der Längsrichtung voneinander beabstandete Saugkörperkomponenten (12c) vorgesehen werden.

8. Vorrichtung (46, 46a) zum Herstellen von Laminatabschnitten (51) nach einem Verfahren nach einem oder mehreren der vorstehenden Ansprüche mit einer Zuführeinrichtungen für die erste Bahn (40) und für die zweite Bahn (41) und mit einer Fügeeinrichtung (48) zum Befestigen der zweiten Bahn auf der ersten Bahn **dadurch gekennzeichnet, dass** in Maschinenrichtung M vor der Fügeeinrichtung (48) eine Einfalteinrichtung (75, 75a) für die erste Bahn (40) vorgesehen ist zum Einfalten der ersten Bahn (40) quer zur Bahnebene und quer zur Maschinenrichtung M, **dadurch gekennzeichnet, dass** die Vorrichtung eine zylinderförmige Anordnung (76, 176) umfasst, und dass die Einfalteinrichtung (75a) 2-9 hintereinander angeordnete Endloseinziehbänder (79a, 79b, 79c, 79d, 79e) aufweist und dass die Endloseinziehbänder (79a, 79b, 79c, 79d, 79e) auf einer Kreisbahn hintereinander angeordnet sind, derart, dass sie die zylinderförmigen Anordnung (176) im Wesentlichen bilden und dass die zylinderförmige Anordnung (176) einen Drehkörper (176a) umfasst, an dessen Mantelfläche die Endloseinziehbänder (79a, 79b, 79c, 79d, 79e) angeordnet sind.

9. Vorrichtung nach Anspruch 8, **dadurch gekennzeichnet, dass** die Einfalteinrichtung (75a) 3-8, weiter insbesondere 4-6 hintereinander angeordnete Endloseinziehbänder (79a, 79b, 79c, 79d, 79e) aufweist.

10. Vorrichtung nach Anspruch 8 oder 9, **dadurch gekennzeichnet, dass** die Endloseinziehbänder (79a, 79b, 79c, 79d, 79e) in Umfangsrichtung der zylinderförmigen Annordnung (176) voneinander beabstandet angeordnet sind, so dass zwischen zwei aufeinanderfolgenden Endloseinziehbändern überschüssiges Material der ersten Bahn aus der Bahnebene und innerhalb der zylinderförmigen Anordnung (176) verbringbar ist.

## Claims

1. Method for producing laminate portions (51) for disposable medical articles (2, 2a, 90) such as absorbent incontinence products or surgical drapes or surgical gowns or wound coverings, wherein the laminate portions (51) comprise a first component (52), which is made from a sheet material such as a nonwoven or film and has a first length L1, and a further, second component (53), which is made from a sheet material such as a nonwoven or film and has a second length L2, wherein L2 < L1, and wherein the second component (53) is arranged around the full periphery within the contour of the first component (52), and wherein a first endless web (40) of a first sheet material is conveyed in a longitudinal direction corresponding to the machine direction M, and wherein a second web (41) of a second sheet material is conveyed in the longitudinal direction and wherein the second web (41) has weakening lines (50) arranged substantially transversely with respect to the longitudinal direction or the weakening lines (50) are created in the course of producing the laminate portions (51), and the first web (40) is folded in transversely with respect to the plane of the web and transversely with respect to the machine direction M, and wherein, after the first web (40) has been folded in, the second web (41) is applied and joined to the first web (40) in such a way that the second web (41) is arranged within the longitudinal edges of the first web (40), and then the composite of the first web (40) and the second web (41) is accelerated in the machine direction M to form a laminate web (49) and folded in in the process, wherein the second web (41) is severed along the weakening lines (50) and as a result longitudinal portions (100) of the second web are spaced apart from one another and wherein, to form the laminate portions (51), the laminate web (49) is separated substantially transversely with respect to the machine direction M, **characterized in that** the first web is folded in such that the first web (40) is fed to a cylindrical assembly (176), wherein the first web is conveyed at a web speed v3 in a first acceleration region in the cylindrical assembly (176), and **in that** after this the first web is slowed down to a second web speed v5 in a deceleration region adjoining the first acceleration region in the machine direction, and **in that** the resultingly accumulating excess material of the first web (40) is temporarily brought into a respective cavity underneath a folding-in opening (74a) in the cylindrical assembly and thus radially displaced and folded out of the plane of the web, and **in that**, after being fed to the cylindrical assembly (176), the first web (40) is grasped by an endless drawing-in belt (79a), driven at the speed v2, in the first acceleration region of the cylindrical assembly (176), wherein v2 > 0 m/s.

2. Method according to Claim 1, **characterized in that** an endless drawing-in belt (79b) which conveys the first web (40) is driven at a slower speed in the deceleration region than the endless drawing-in belt (79a) in the first acceleration region, with the result that the first web (40) is slowed down.

3. Method according to Claim 1 or 2, **characterized in that** the cylindrical assembly (176) comprises a cylindrical rotational element (76a, 76b), on the circumferential surface of which the endless drawing-in belts (79a, 79b, 79c, 79d, 79e) are arranged.

4. Method according to one of Claims 1-3, **characterized in that** a.) the first web (40) comprises or is made from a nonwoven material and the second web (41) comprises or is made from a film material, or b.) the first web (40) comprises or is made from a film material and the second web (41) comprises or is made from a nonwoven material.

5. Method according to one of the preceding claims, **characterized in that** the second web (41) comprises or is made from a material that absorbs bodily fluids.

6. Method according to one of the preceding claims, **characterized in that** the longitudinal edges of the second component (53) are arranged flush with the longitudinal edges of the first component (52) in the laminate portions (51).

7. Method according to one of the preceding claims, **characterized in that** absorbent-element components (12c) that are spaced apart in the longitudinal direction are provided on the laminate web (49).

8. Apparatus (46, 46a) for producing laminate portions (51) by a method according to one or more of the preceding claims, having a feed device for the first web (40) and a feed device for the second web (41) and having a joining device (48) for fastening the second web to the first web, **characterized in that** a folding-in device (75, 75a) for the first web (40) is provided upstream of the joining device (48) in the machine direction M for the purpose of folding in the first web (40) transversely with respect to the plane of the web and transversely with respect to the machine direction M, **characterized in that** the apparatus comprises a cylindrical assembly (76, 176), and **in that** the folding-in device (75a) has 2-9 endless drawing-in belts (79a, 79b, 79c, 79d, 79e) arranged one behind another and **in that** the endless drawing-in belts (79a, 79b, 79c, 79d, 79e) are arranged one behind another on a circular path in such a way that they substantially form the cylindrical assembly (176) and **in that** the cylindrical assembly (176) comprises a rotational element (176a), on the lateral surface of which the endless drawing-in belts (79a, 79b, 79c, 79d, 79e) are arranged.

9. Apparatus according to Claim 8, **characterized in that** the folding-in device (75a) has 3-8, further particularly 4-6, endless drawing-in belts (79a, 79b, 79c, 79d, 79e) arranged one behind another.

10. Apparatus according to Claim 8 or 9, **characterized in that** the endless drawing-in belts (79a, 79b, 79c, 79d, 79e) are arranged spaced apart in the circumferential direction of the cylindrical assembly (176), with the result that, between two endless drawing-in belts that follow one another, excess material of the first web can be brought out of the plane of the web and inside the cylindrical assembly (176).

## Revendications

1. Procédé de fabrication de sections stratifiées (51) pour des articles médicaux à usage unique (2, 2a, 90) tels que des produits absorbants pour l'incontinence ou des couvertures chirurgicales ou des vêtements chirurgicaux ou des couvertures de blessures, les sections stratifiées (51) comprenant un premier composant (52) formé d'un matériau plat tel qu'un non-tissé ou un film, ayant une première longueur L1, et un autre deuxième composant (53) formé d'un matériau plat tel qu'un non-tissé ou un film, ayant une deuxième longueur L2, avec L2 < L1, et le deuxième composant (53) étant agencé entièrement à l'intérieur du contour du premier composant (52), et une première bande sans fin (40) d'un premier matériau plat étant transportée dans une direction longitudinale qui correspond à la direction de la machine M, et une deuxième bande (41) d'un deuxième matériau plat étant transportée dans la direction longitudinale, et la deuxième bande (41) présentant des lignes d'affaiblissement (50) agencées essentiellement transversalement à la direction longitudinale, ou les lignes d'affaiblissement (50) étant créées au cours de la fabrication des sections stratifiées (51), et la première bande (40) étant pliée transversalement au plan de la bande et transversalement à la direction de la machine M, et, après ce pliage de la première bande (40), la deuxième bande (41) étant appliquée sur la première bande (40) et assemblée, de telle sorte que la deuxième bande (41) est agencée à l'intérieur des bords longitudinaux de la première bande (40), puis le composite de la première bande (40) et de la deuxième bande (41) est accéléré dans la direction de la machine M pour former une bande stratifiée (49) et est ainsi déplié, la deuxième bande (41) étant séparée le long des lignes d'affaiblissement (50) et des sections longitudinales (100) de la deuxième bande étant ainsi espacées les unes des autres et la bande stratifiée (49) étant individualisée essentiellement transversalement à la direction de la machine M pour former les sections stratifiées (51), **caractérisé en ce que** le pliage de la première bande est réalisé de telle sorte que la première bande (40) est amenée à un agencement cylindrique (176), la première bande étant transportée dans l'agencement cylindrique (176) dans une première zone d'accélération à une vitesse de bande v3, et que la première bande est ensuite freinée à une deuxième vitesse de bande v5 dans une zone de décélération contigüe dans la direction de la machine à la première zone d'accélération, et que l'excès de matériau de la première bande (40) qui en résulte est transféré temporairement dans une cavité respective en dessous d'une ouverture de pliage (74a) de l'agencement cylindrique et est ainsi déplacé radialement hors du plan de la bande et plié, et que la première bande (40), après avoir été amenée à l'agencement cylindrique (176), est saisie par une bande d'alimentation sans fin (79a) entraînée à la vitesse v2 dans la première zone d'accélération de l'agencement cylindrique (176), avec v2 > 0 m/s.

2. Procédé selon la revendication 1, **caractérisé en ce qu'**une bande d'alimentation sans fin (79b) transportant la première bande (40) est entraînée dans la zone de décélération à une vitesse inférieure à celle de la bande d'alimentation sans fin (79a) dans la première zone d'accélération, de telle sorte que la première bande (40) est freinée.

3. Procédé selon la revendication 1 ou 2, **caractérisé en ce que** l'agencement cylindrique (176) comprend un corps cylindrique rotatif (76a, 76b) sur la surface périphérique duquel sont agencées les bandes d'alimentation sans fin (79a, 79b, 79c, 79d, 79e).

4. Procédé selon l'une quelconque des revendications 1 à 3, **caractérisé en ce que** a.) la première bande (40) comprend un matériau non tissé ou est formée à partir de celui-ci et la deuxième bande (41) comprend un matériau en film ou est formée à partir de celui-ci ou b.) la première bande (40) comprend un matériau en film ou est formée à partir de celui-ci et la deuxième bande (41) comprend un matériau non tissé ou est formée à partir de celui-ci.

5. Procédé selon l'une quelconque des revendications précédentes, **caractérisé en ce que** la deuxième bande (41) comprend un matériau absorbant les fluides corporels ou est formée à partir de celui-ci.

6. Procédé selon l'une quelconque des revendications précédentes, **caractérisé en ce que**, dans les sections stratifiées (51), les bords longitudinaux du deuxième composant (53) sont agencés en affleurement des bords longitudinaux du premier composant (52).

7. Procédé selon l'une quelconque des revendications précédentes, **caractérisé en ce que** des composants de corps aspirant (12c) espacés les uns des autres dans la direction longitudinale sont prévus sur la bande stratifiée (49).

8. Dispositif (46, 46a) pour la fabrication de sections stratifiées (51) par un procédé selon une ou plusieurs des revendications précédentes avec un dispositif d'amenée pour la première bande (40) et pour la deuxième bande (41) et avec un dispositif d'assemblage (48) pour la fixation de la deuxième bande sur la première bande, **caractérisé en ce qu'**un dispositif de pliage (75, 75a) pour la première bande (40) est prévu avant le dispositif d'assemblage (48) dans la direction de la machine M pour plier la première bande (40) transversalement au plan de la bande et transversalement à la direction de la machine M, **caractérisé en ce que** le dispositif comprend un agencement cylindrique (76, 176), et **en ce que** le dispositif de pliage (75a) présente 2 à 9 bandes d'alimentation sans fin (79a, 79b, 79c, 79d, 79e) agencées les unes derrière les autres et **en ce que** les bandes d'alimentation sans fin (79a, 79b, 79c, 79d, 79e) sont agencées les unes derrière les autres sur une trajectoire circulaire, de telle sorte qu'elles forment essentiellement l'agencement cylindrique (176) et **en ce que** l'agencement cylindrique (176) comprend un corps rotatif (176a) sur la surface d'enveloppe duquel sont agencées les bandes d'alimentation sans fin (79a, 79b, 79c, 79d, 79e).

9. Dispositif selon la revendication 8, **caractérisé en ce que** le dispositif de pliage (75a) présente 3 à 8, plus particulièrement 4 à 6 bandes d'alimentation sans fin (79a, 79b, 79c, 79d, 79e) agencées les unes derrière les autres.

10. Dispositif selon la revendication 8 ou 9, **caractérisé en ce que** les bandes d'alimentation sans fin (79a, 79b, 79c, 79d, 79e) sont agencées espacées les unes des autres dans la direction périphérique de l'agencement cylindrique (176), de telle sorte qu'entre deux bandes d'alimentation sans fin successives, le matériau en excès de la première bande peut être transféré hors du plan de la bande et à l'intérieur de l'agencement cylindrique (176).
